# EUROPEAN PATENT APPLICATION

(11) **EP 0 629 405 A1**
(43) Date of publication of application: **21.12.1994**
(21) Application number: 94304450.3
(22) Date of filing: 20.06.1994
(51) Int. Cl.: A61K 31/59

(54) **Agent for inhibiting a decrease in bone mass in renal osteodystrophy**

(30) Priority: 21.06.1993 JP 172032/93
(71) Applicant: KUREHA CHEMICAL INDUSTRY CO., LTD., Chuo-ku Tokyo 103 (JP)
(72) Inventor: Murayama, Hisashi, Tokyo (JP); Kumagai, Miki, Tokyo (JP); Taniguchi, Nobuyuki, Nerima-ku, Tokyo (JP)
(74) Representative: Spencer, Michael David, Dr.

(57) **Abstract**

A pharmaceutical composition comprising 24, 25-dihydroxycholecalciferol is useful for the remedy or prophylaxis of disorders associated with renal osteodystrophy. The active compound, 24, 25-(OH)₂-D₃, alone unexpectedly not only improves bone quality but also prohibits bone mass decreases in renal osteodystrophy.

## Description

### Field of the Invention

This invention relates to an agent for alleviating symptom in renal osteodystrophy, which comprises an effective amount of 24, 25-dihydroxycholecalciferol (hereinafter sometimes referred to as 24, 25-(OH)₂-D₃ or the active compound of the present invention) in admixture with a pharmaceutically acceptable carrier. In particular, the agent is useful in prohibiting a decrease in bone mass in renal osteodystrophy.

### Background of the Invention

A renal dysfunction leads to abnormal calcium and phosphate metabolism and to aberrant vitamin D activation, which induce renal osteodystrophy.

Renal osteodystrophy is histologically classified into osteomalacia type, osteitis fibrosa type, mixed type, low turnover type, and mild type, according to the pathogenesis and histological evaluation. It is thought that the renal osteodystrophy is a disease wherein bone quality changes, in contrast to osteoporosis, wherein bone mass decreases. From the histological evaluation of bone biopsy specimens, even though there are some differences in the degree of bone lesion, it is thought that there are abnormalities of bone in almost all bone histological specimens from renal insufficient patients.

Further, a recent technological advance using photon-energy of single wavelength for a quantitative measurement of bone mass enables the assessment of bone mass quantitatively.

Since it is possible to measure quantitatively bone mass of the whole bone by means of such photoenergetic method in contrast to a conventional observation by an X-ray technique or a histological evaluation on bone tissues, this method is clinically quite useful. Consequently, it has been revealed that bone mass in renal osteodystrophy patients, who have increased risk of bone fractures decreases. However, the pathogenesis thereof is still indistinct with an unclear relation to biochemical markers or hormones related with bone metabolism. Therefore, it may take time to solve these problems through future extensive researches.

In order to treat diseases in a renal insufficient patient, vitamin D has been administered, together with dieting and dialysis to control blood phosphate and to prevent hypocalcemia. Specifically, vitamin D₃ or its metabolite is effective in preventing hypocalcemia by promoting calcium absorption from the intestine. Furthermore, vitamin D₃ or its metabolite has been widely applied to renal insufficient patients, especially for the case where secondary hyperparathyroidism is observed because it has an inhibitive action against parathyroid hormone production.

However, no therapeutic drug has been found that, when used alone, is capable of remedying bone mass and inhibiting a decrease in bone mass without any side effect when it is used alone. It has also been eagerly desired to develop a novel safe drug capable of remedying bone quality and inhibiting a decrease in bone mass without an adverse action in treatment of renal osteodystrophy.

As aforementioned, it has not yet been established how to remedy the change of bone quality and the decrease of bone mass in renal osteodystrophy, nor has the pathogenesis of renal osteodystrophy been elucidated.

The present inventors have made extensive investigations on the development of therapeutic drugs capable of inhibiting the bone mass decrease by use of experimental animal models wherein the bone quality is deteriorated and the bone mass decreases as renal insufficiency progresses. As a result, the present inventors have discovered that 24, 25-(OH)₂-D₃, which is present in the healthy human body and proved to be safe, is effective in remedying bone quality and inhibiting a decrease in bone mass in renal osteodystrophy upon its single application. Consequently, the present inventors have succeeded in inventing the novel therapeutic drugs for renal osteodystrophy on the basis of its discovery.

### Summary of the Invention

The present invention relates to a pharmaceutical composition for alleviating or preventing symptom in renal osteodystrophy, which comprises an effective amount of 24, 25-dihydroxycholecalciferol (or briefly referred to as 24, 25-(OH)₂-D₃) in admixture with a pharmaceutically acceptable carrier. In particular, the pharmaceutical composition is useful in remedying bone quality and inhibiting a decrease in bone mass in renal osteodystrophy upon its single application. In another aspect of the present invention, there is provided a method for the treatment or prophylaxis of renal osteodystrophy in a mammalian host, which comprises administering an effective amount of 24, 25-(OH)₂-D₃ to said host suffering from renal osteodystrophy. The present invention relates to a use of an effective amount of 24, 25-(OH)₂-D₃ for preparing a pharmaceutical composition for alleviating or preventing at least one symptom in renal osteodystrophy.

Further, the present invention relates to an agent for alleviating or preventing disorders accompanied with renal osteodystrophy, which comprises 24, 25-(OH)₂-D₃ alone as an active ingredient.

### Detailed Description of Preferred Embodiments

The active ingredient, 24, 25-(OH)₂-D₃ is disclosed in documents such as Pharmacia (Japan), 10, 319 - 322, (1974). It is also known that it has an anti-hypercalcemic action (Japanese Patent Publication No. 21329/1987), anti-hyperphosphatemic action (Japanese Patent Publication No. 13202/1990), Mg regulation action (Japanese Patent Publication No. 21330/1987), anti-osteoporotic action (Japanese Patent Publication No. 32208/1989), anti-uremic action (Japanese Patent Publication No. 53847/1992), and algesia-alleviation action (Japanese Patent Publication No. 72206/1992, 72207/1992). Although it has been reported that it improves osteohistological bone quality parameters in a renal osteodystrophy patient under a combined use with 1α -hydroxycholecalciferol (1α -(OH)-D₃ (Japanese Patent Application Laid Open No. 31722/1989), it is still not disclosed therein whether it alone has actions to remedy bone quality and to inhibit a decrease in bone mass. The active compound, 24, 25-(OH)₂-D₃, includes isomers, 24R, 25-(OH)₂-D₃ and 24S, 25-(OH)₂-D₃. For the composition of the present invention, 24R, 25-(OH)₂-D₃, 24S, 25-(OH)₂-D₃ or a racemate thereof can be used and specifically 24R, 25-(OH)₂-D₃ is preferable.

The active compound of the present invention is tested for acute toxicity and the resultant data is shown as follows:

The active compound of the present invention, 24R, 25-(OH)₂-D₃ is admixed with a sesame oil (containing 0.5 % ethanol) and the mixture is orally administered to 4 groups of female and male ICR mice (each group has 5 mice) (body weight: male 29 to 33 g, female 22 to 29 g). Two weeks later, toxic symptom is observed. From death rates for 2 weeks, LD₅₀ (lethal dose, 50% mortality) is evaluated as follows:
male 215 mg/kg and
female 253 mg/kg.

The active compound is considerably safer, in contrast to vitamin D₃ that has been used so far in the prior medical field, its metabolite, and the aforementioned 1 α -(OH)-D₃.

Although it can be investigated on an action of the active compound to bone quality and bone mass by means of a renal insufficient animal model, the animal model wherein renal insufficiency induces abnormal bone quality and low bone mass very close to clinical renal osteodystrophy is unknown in public in the prior arts.

In view of these circumstances, the present inventors have made extensive investigations to develop novel rat models wherein renal insufficiency induces abnormal bone quality and low bone mass similar to clinical renal osteodystrophy. As a result, the present inventors have succeeded in obtaining an animal model with such diseases. Thus, after 7/8 of kidney arteries were ligated, animals endurable even for a long breeding term with a mild renal insufficiency were selected and carefully bred for 6 months to establish an animal model with renal osteodystrophy with accompanying abnormal bone quality and low bone mass.

Consequently, the active compound of the present invention can be studied on its activity in remedying bone quality and in inhibiting a bone mass decrease by means of the experimental animal model thus obtained. Also, the animal models are predictive of activity in humans.

Different from a sham-operated control group, the renal insufficient animal raises a bone disease wherein a bone mass decrease is observed. Upon administration of the active compound according to the present invention at the dose level ranging from 0.1 to 5 µg/kg, the experimental animal can remedy its bone histological characteristics, retain bone mass to an extent equivalent to that in the sham-operated control group and prevent a bone mass decrease, relative to a non-dose group.

The pharmaceutical composition according to the present invention for inhibiting a decrease in bone mass in renal osteodystrophy can be applied orally, subcutaneously, or intravenously. The active compound according to the present invention, 24, 25-dihydroxycholecalciferol, can be admixed with a pharmaceutically acceptable ingredient or carrier to form a pharmaceutical composition which may be formulated to various dosage forms such as tablets, powders, granules, suppositories, capsules, soft capsules, alcoholic formulations, oily solutions and aqueous suspensions.

Examples of oily solvents for the above-mentioned formulations may include a triglyceride ester of neutral fatty acid, corn oil, cotton seed oil, peanut oil, fish liver oil, sesame oil, an oily ester, etc. Cocoa oil, glycerin, etc. is also preferably used. Other carriers or ingredients may include lactose, starch, talc, magnesium stearate, sorbic acid, and sorbate, or its derivatives, alcohol, physiological saline, surface-active agents, and antioxidants, etc., which can be used in admixture with the active compound of the invention.

24, 25-dihydroxycholecalciferol can be used as the only active ingredient in a pharmaceutical composition. Preferably, 24, 25-dihydroxycholecalciferol is the only renal osteodystrophy active ingredient (i.e., only active ingredient effective in preventing or alleviating renal osteodystrophy symptoms) used in a treatment. However, other active ingredients can be present, with the proviso that none of 1α -(OH)-D₃, 1, 25-dihydroxycholecalciferol or dihydrotachysterol is adminidtered with the 24, 25-(OH)₂-D₃.

The pharmaceutical composition preferably comprises the active compound of the invention in an amount ranging from 0.00002 to 4 wt % per single dosage form. The active compound may be administered to an adult human target with an amount ranging from 0.1 to 100,000 µg, and preferably from 10 to 60,000 µg, per day. Administeration amounts of at least 50 µg per day of 24, 25-(OH)₂-D₃ are also suitable. Dosage levels may range from 0.02 to 2,000 µg/kg on a daily basis. Dosage forms such as discussed above may comprise 0.02 to 4,000 µg of 24, 25-(OH)₂-D₃.

### Working Examples

The following working examples are intended to illustrate the invention in further detail and should by no means be construed as limiting the scope of the invention.

### Working Example 1

After male SD rats with 7/8 of their kidney arteries ligated at 11 weeks old are bred for 8 weeks, 24R, 25-(OH)₂-D₃ and 24, 25-(OH)₂-D₃ (a racemate of 24R and 24S isomers) are administered orally to the rat for 4 months, respectively.

A solution of sesame oil (the Pharmacopoeia of Japan) containing 0.5 % ethanol is utilized as the solvent. The test is conducted by using 5 groups each of which consists of 6 to 10 rats as follows:
I. sham-operated control group (normal control group, solvent administration) n=8
II. solvent administration control group n=6
III. 24R, 25-(OH)₂-D₃ 0.1 µg/kg n=6
IV. 24R, 25-(OH)₂-D₃ 5.0 µg/kg n=10
V. 24, 25-(OH)₂-D₃ 5.0 µg/kg n=10

After administration, blood is anesthetically collected and applied to a biochemical serum analysis. After femur is resected, mineral content of femur is measured by means with equipment capable of measuring bone mass (single photon absorptiometry; SPA). Further, the bone tissue sample is stained by Villaneuva's bone staining after slicing, followed by observation under a microscope.

The serum creatinine level rose by ligation of 7/8 of renal arteries (the sham-operated control group versus the ligation-operated control group: 0.44± 0.05 vs 1.02± 0.38 mg/dl, p<0.001). Consequently, it is clear that the ligation of 7/8 of the renal arteries induced renal insufficiency in the experimental animals, wherein the serum phosphate level rose slightly while the serum calcium level was unchanged and the serum parathyroid hormone level rose significantly. From the observation of the bone tissue specimens, an osteitis fibrosa is found in the ligation-operated control group. Therefore, it is concluded that bone disorders associated with renal osteodystrophy is reproduced.

Further, in the measurement of bone mineral content, a bone mass decrease is found (the sham-operated control group versus the ligation-operated control group: 0.284± 0.023 vs 0.259± 0.017 g/cm², p<0.05). Thus, the experimental animal model raised not only abnormal bone quality of renal osteodystrophy, as known, but also shows lower bone mass. Therefore, it is clear that the animal model is useful for an investigation on drug actions against the subject symptoms.

The groups which are given 5.0 µg/kg of 24, 25-(OH)₂-D₃ or 0.1µg/kg or 5.0 µg/kg of 24R, 25-(OH)₂-D₃ depress a decrease in bone mass (0.282± 0.025, 0.291± 0.032, and 0.292 ± 0.019* g/cm², respectively; * p<0.01) and kept bone mass similar to those in the sham-operated control group. Specifically, bone mass is kept in the group of 24R, 25-(OH)₂-D₃ 5.0µg/kg administration in significant contrast to the only solvent administration control group. Further, through the histological observation of bone tissue specimens, none of bone disorders including osteitis fibrosa, which are observed in renal osteodystrophy, are found in the treated groups. As for the treated group, the bone quality is improved and the histological feature of bone specimens is similar to that in the sham-operated control group.

### Working Example 2

24R, 25-(OH)₂-D₃ (200 mg) is dissolved in ethanol (5 ml) and the resultant mixture is added to a sesame oil (2 kg) followed by dissolution. Highly pure argon gas is bubbled through the sesame oil solution to remove oxygen present therein.

Soft-capsules are prepared by a conventional method using a soft-capsule manufacturing machine to contain 10µg of 24R, 25-(OH)₂-D₃ per capsule.

Shell composition (part by weight in the final product)
- gelatin: 10 parts by weight
- glycerin: 2 parts by weight
- preservative:
- (ethylparaben): 0.05 parts by weight
- titanium white: 0.2 parts by weight
- water: 0.2 parts by weight

The embodiments of the invention also include:
1. A method for alleviating or preventing at least one symptom in renal osteodystrophy, which comprises administering to a mammalian host suffering from renal osteodystrophy an independently effective amount of 24, 25-dihydroxycholecalciferol to relieve said host of said at least one symptom.
2. A method for alleviating or preventing at least one symptom in renal osteodystrophy according to Claim 11, which comprises administering to a mammalian host suffering from renal osteodystrophy an independently effective amount of at least 50 µg per day of 24, 25-dihydroxycholecalciferol to relieve said host of said at least one symptom.
3. A method according to Item 1, wherein the at least one symptom is a defect of bone quality in renal osteodystrophy.
4. A method according to Item 1, wherein the at least one symptom is a defect of bone mass in renal osteodystrophy.
5. A method according to Item 1, wherein the at least one symptom is a decrease in bone mass in renal osteodystrophy.
6. A method according to Item 1, wherein the 24, 25-dihydroxycholecalciferol consists essentially of 24R, 25-dihydroxycholecalciferol.
7. A method according to Item 1, wherein the 24, 25-dihydroxycholecalciferol is administered daily at a dose level ranging from 0.02 to 2,000µg/kg.
8. A method according to Item 1, wherein the 24, 25-dihydroxycholecalciferol is administered orally, subcutaneously or intravenously.
9. A method for alleviating or preventing at least one symptom in renal osteodystrophy, which comprises administering to a mammalian host suffering from renal osteodystrophy an independently effective amount of 24, 25-dihydroxycholecalciferol to relieve said host of said at least one symptom, with the proviso that none of 1α -dihydroxycholecalciferol, 1, 25-dihydroxycholecalciferol or dihydrotachysterol are administered with the 24, 25-dihydroxycholecalciferol in said method.
10. A method for alleviating or preventing at least one symptom in renal osteodystrophy, which comprises administering to a mammalian host suffering from renal osteodystrophy a pharmaceutical composition whose pharmaceutically active component consists essentially of an effective amount of 24, 25-dihydroxycholecalciferol to relieve said host of said at least one symptom.
11. A method according to Item 10, wherein said pharmaceutical composition is in a dosage form comprising from 0.02 to 4000 µg of 24, 25-dihydroxycholecalciferol and a a pharmaceutically effective carrier.
12. A method according to Item 11, wherein said dosage form is a soft capsule.
13. A method according to Item 10, wherein said pharmaceutical composition is in a dosage form comprising 24, 25-dihydroxycholecalciferol in an amount of from 0.00002 to 4 wt % of said dosage form and a pharmaceutically effective carrier.

## Claims

1. A pharmaceutical composition comprising an active ingredient comprising an amount of 24, 25-dihydroxycholecalciferol effective to alleviate or prevent at least one symptom in renal osteodystrophy in admixture with a pharmaceutically acceptable carrier.

2. The pharmaceutical composition according to Claim 1, wherein the amount of 24, 25-dihydroxycholecalciferol is independently effective to alleviate or prevent symptoms of renal osteodystrophy.

3. The pharmaceutical composition according to Claim 1, wherein the amount of 24, 25-dihydroxycholecalciferol is effective to alleviate or prevent the defect of bone quality in renal osteodystrophy.

4. The pharmaceutical composition according to Claim 1, wherein the amount of 24, 25-dihydroxycholecalciferol is effective to alleviate or prevent the defect of bone mass in renal osteodystrophy.

5. The pharmaceutical composition according to Claim 1, wherein the amount of 24, 25-dihydroxycholecalciferol is effective to inhibit a decrease in bone mass in renal osteodystrophy.

6. The pharmaceutical composition according to Claim 1, wherein the 24, 25-dihydroxycholecalciferol consists essentially of 24R, 25-dihydroxycholecalciferol.

7. The pharmaceutical composition according to Claim 1, wherein said pharmaceutical composition is in a dosage form comprising from 0.02 to 4000 µg of 24, 25-dihydroxycholecalciferol.

8. The pharmaceutical composition according to Claim 7, wherein said dosage form is a soft capsule.

9. The pharmaceutical composition according to Claim 1, wherein said pharmaceutical composition is in a dosage form comprising 24, 25-dihydroxycholecalciferol in an amount of from 0.00002 to 4 wt % of said dosage form.

10. A pharmaceutical composition for alleviating or preventing at least one symptom in renal osteodystrophy, whose pharmaceutically active component consists essentially of an effective amount of 24, 25-dihydroxycholecalciferol.

11. A use of an effective amount of 24, 25-dihydroxycholecalciferol for preparing a pharmaceutical composition for alleviating or preventing at least one symptom in renal osteodystrophy.

12. A use of Claim 11, wherein the pharmaceutical composition has an independently effective amount of 24, 25-dihydroxycholecalciferol.
